# EUROPEAN PATENT SPECIFICATION

(11) **EP 0 662 475 B1**
(45) Date of publication and mention of the grant of the patent: **06.05.1998**
(21) Application number: 94117932.7
(22) Date of filing: 14.11.1994
(51) Int. Cl.: C07D 263/42, C07D 413/04, C07C 255/42, C07D 333/24

(54) **Method for the preparation of 2-perfluoroalkyl-3-oxazolin-5-one**
Verfahren zur Herstellung von 2-Perfluoroalkyl-3-oxazolin-5-one
Procédé pour la préparation de dérivés de 2-perfluoroalkyle-3-oxazolin-5-one

(30) Priority: 30.12.1993 US 175845; 30.12.1993 US 175822
(43) Date of publication of application: 12.07.1995
(73) Proprietor: AMERICAN CYANAMID COMPANY, WayneNew Jersey 07470 (US)
(72) Inventor: Kameswaran, Venkataraman, Princeton Junction,New Jersey 08550 (US)
(74) Representative: Wächtershäuser, Günter, Prof. Dr.

(56) References cited:
- EP-A- 0 595 130
- US-A- 5 030 735
- TETRAHEDRON, vol. 46,no. 4, 1990 BERGMAN 'sythesis of chrysogine'
- J.ORG.CHEM., vol. 43,no. 22, 1978 BENAGES '2-Chloroacyrlonitrile as a Cyclodipolarohile in 1,3-Cycloadditions.'
- SYNTHESIS, vol. 11, 1972 POUPAERT 'N-Acyl-alpha-aminonitriles in the Pinner reaction.'

## Description

Arylpyrrole carbonitrile compounds and derivatives thereof are highly effective insecticidal, acaricidal and nematocidal agents. In particular 2-aryl-5-trifluoromethylpyrrole-3-carbonitrile compounds and their derivatives have been found to have a broad spectrum of activity at very low rates of application with effectiveness against resistant species. U.S. Patent 5,030,735 describes methods to prepare said pyrrole compounds on a manufacturing scale and includes the 1,3-dipolar cycloaddition of the appropriate 3-oxazolin-5-one with 2-chloroacrylonitrile. Heretofore the 3-oxazolin-5-one key intermediate has been prepared through the appropriate phenylglycine compound in a 4 step synthetic route starting from the preceding aminonitrile.

It is an object of this invention to provide a facile and efficient synthesis of 2-perfluoroalkyl-3-oxazolin-5-one in 2 steps from the aminonitrile precursor.

In addition is an object of this invention to provide perfluoroalkanoyl aminonitrile compounds useful in preparing 2-perfluoroalkyl-3-oxazoline-5-one.

It is a further object of this invention to provide a convenient source of a key intermediate in the manufacture of insecticidal, acaricidal and nematocidal arylpyrrole compounds.

### DESCRIPTION OF THE INVENTION

There is provided a method for the preparation of a 2-perfluoroalkyl-3-oxazolin-5-one compound of formula II 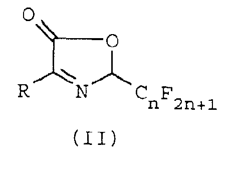 wherein
n is an integer of 1, 2, 3, 4, 5, 6, 7 or 8;
R is 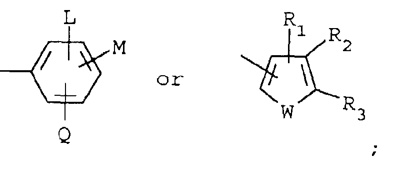 L is hydrogen or halogen;
M and Q are each independently hydrogen, halogen, CN, N0₂, C₁-C₄alkyl, C₁-C₄haloalkyl, C₁-C₄alkoxy, C₁-C₄haloalkoxy, C₁-C₄alkylthio, Cₗ-C₄alkylsulfinyl or when M and Q are on adjacent positions they may be taken together with the carbon atoms to which they are attached to form a ring in which MQ represents the structure 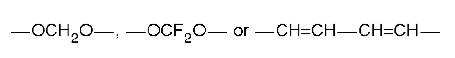 with the proviso that at least one of L, M and Q must be other than hydrogen;
R₁, R₂ and R₃ are each independently hydrogen, halogen, N0₂, CHO or R₂ and R₃ may be taken together with the atoms to which they are attached to form a ring in which R₂R₃ is represented by the structure 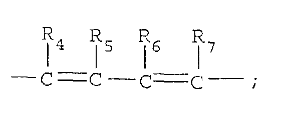
R₄, R_{5;} R₆ and R₇ are each independently hydrogen, halogen, CN or NO₁: and W is O or S which comprises reacting an aminonitrile of formula lll 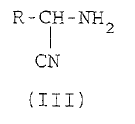 wherein R is as described above with a perfluoro-acylating agent of formula IV 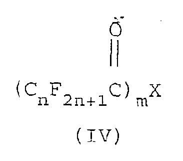 wherein m is an integer of 1 or 2, X is OR₁, Cl or O and R₁ is hydrogen or C₁-c₆alkyl with the proviso that when X is O, m must be 2 and when X is Cl or OR₁, then m must be 1 in the presence of a solvent, optionally in the presence of a base, to form a perfluoro-alkanoyl aminonitrile intermediate of formula I 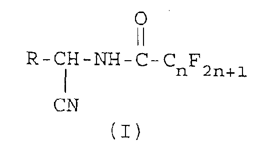 and cyclizing the formula I intermediate in the presence of an acid and at least one molar equivalent of water. Preferred processes are defined in claims 2 and 3.

There is also provided a perfluoroalkanoyl aminonitrile intermediate of formula I 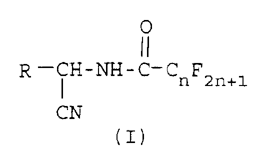 wherein
n is an integer of 1, 2, 3, 4, 5, 6, 7 or 8;
R is 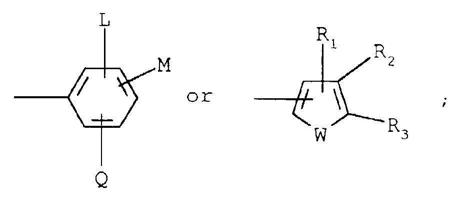 L is hydrogen or halogen;
M and Q are each independently hydrogen, halogen, CN, N0₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure 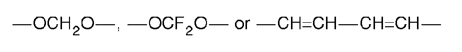 with the proviso that at least one of L, M and Q must be other than hydrogen;
R₁, R₂ and R₃ are each independently hydrogen, halogen, N0₂, CHO or R₂ and R₃ may be taken together with the atoms to which they are attached to form a ring in which R₂R₃ is represented by the structure 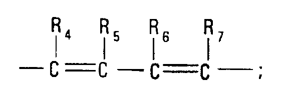
R₄, R₅; R₆ and R₇ are each independently hydrogen, halogen, CN or N0₂; and
W is O or S.

Preferred intermediates are defined in claims 9 and 10.

The 2-perfluoroalkyl-3-oxazolin-5-one compound is a key intermediate in the manufacture of insecticidal, acaricidal and nematicidal pyrrole compounds.

Arylpyrrole compounds, particularly 2-aryl-5-trifluoromethylpyrrole-3-carbonitrile compounds are a new class of highly effective insecticidal, acaricidal and nematocidal agents. A key intermediate in their preparation is the 2-perfluoroalkyl-3-oxazolin-5-one compound of formula ll 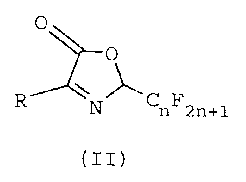 wherein n and R are as described hereinabove. Methods currently known to prepare the formula ll oxazolinone involve the preparation of an appropriate arylglycine compound V via the hydrolysis of the aminonitrile lll . The aminonitrile is obtained via the Strecker synthesis from the appropriate aldehyde precursor(W. L. Matier et al, J. Med. Chem.,1973, 16,901). Protection of the amino group in the aminonitrile III by acetylation to Vl followed by acidic hydrolysis of both the cyano and the protecting groups is required due to the instability of the aminonitrile III under hydrolysis conditions. The thus-obtained glycine V is then trifluoroacetylated to give Vll and cyclized to give the desired oxazolinone ll in 4 steps. The reaction sequence is shown in flow diagram I, wherein R is p-chlorophenyl and n is 1.

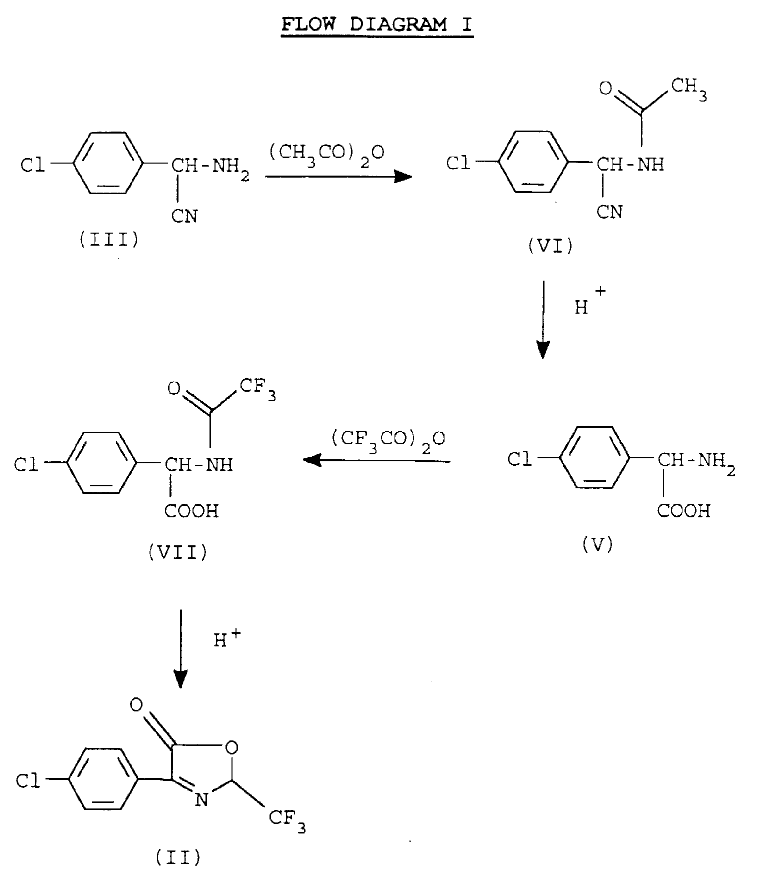

It has now been found that direct perfluoroacylation of the Strecker product lll gives the perfluoroalkanoyl aminonitrile intermediate l which may be readily converted to the desired 2-perfluoroalkyl-3-oxazolin-5-one compound ll. The reaction is shown in flow diagram ll wherein m is 1 or 2, X is Cl, OR₁ or O and R₁ is hydrogen or C₁ -C₆alkyl with the proviso that when X is O, then m must be 2 and when X is Cl or OR₁, then m must be 1.

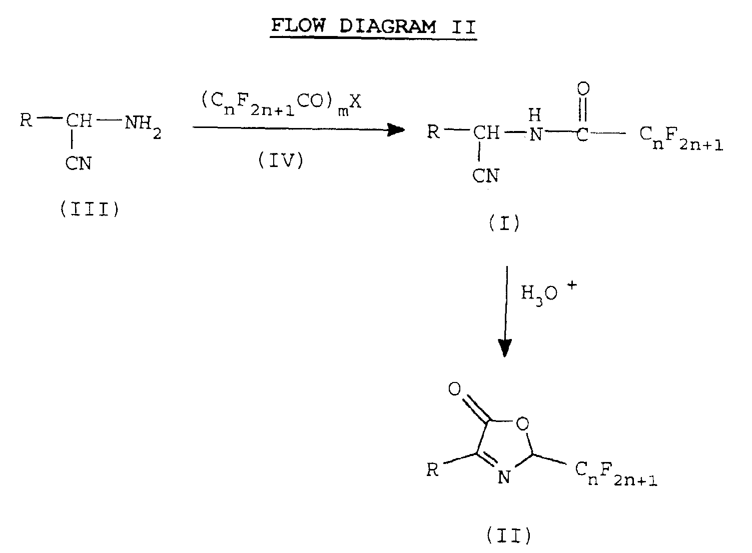

Surprisingly, the perfluoroalkanoyl aminonitrile of formula I may be cyclized in a single step in good yield under aqueous acid conditions to the 2-perfluoroalkyl-3-oxazolin-5-one compound of formula ll.

Advantageously, the desired oxazolinone ll may be obtained in just 2 steps from the Strecker product aminonitrile lll.

Preferred compounds of formula I are those wherein n is 1, 2 or 3, more preferred are those wherein n is 1. Also preferred are those compounds of formula I wherein R is phenyl optionally substituted with one to three halogen, N0₂, C₁-C₄haloal kyl or C₁-C₄haloalkoxy groups.

In accordance with the method of invention, an aminonitrile of formula III is admixed with approximately an equimolar amount of a perfluoro-acylating agent of formula IV in the presence of a solvent, optionally in the presence of a base, to form the perfluoroalkanoyl aminonitrile of formula l The formula I compound is then cyclized in the presence of an aqueous acid to form the formula ll compound, 2-perfluoralkyl-3-oxazolin-5 one.

Solvents suitable for use in the method of invention are aromatic hydrocarbons, or halogenated aromatic hydrocarbons, preferably aromatic hydrocarbons such as toluene, benzene, xylene and the like, more preferrably, toluene.

Acids suitable for use in the method of invention include sulfuric acid, methanesulfonic acid, benzenesulfonic acid, p-toluenesulfonic acid, naphthlenesulfonic acid, fluoroboric acid, boron trifluoride complexes and the like. Boron trifluoride complexes may include BF₃ etherate, BF₃ methanol complex, BF₃ ethanol complex, BF₃ dihydrate and the like. Water may be introduced as a hydrate, i. e. p-toluensulfonic acid monohydrate or as a solute such as 30% - 60% aqueous sulfuric acid.

In actual practice, if a perfluoroacyl chloride, such as trifluoroacetyl chloride, is used as the formula IV reagent, then an equimolar amount of a base may be added as an HCI scavenger. Among the bases which may be used are alkali metal carbonates or bicarbonates or mixtures thereof or tertiary amines. Alkalai metal carbonates such as sodium carbonate or potassium carbonate are contemplated as are bicarbonates such as sodium or potassium bicarbonate.

Tertiary amines suitable for use in the method of invention include any trisubstituted amine know in the art such a trialkylamine, dialkylarylamine, triarylamine, and the like preferably trialkylamine, more prefereably triethylamine.

In order to provide a more clear understanding of the invention, the following examples are set forth below

The terms ¹H, ¹³C and ¹⁹FNMR designate proton, carbon 13 and fluorine 19 nuclear magnetic resonance, respectively

### EXAMPLE 1

Preparation of N-[(p.Chlorophenyl)cyanomethyl].2,2,2.trifl uoroacetamide 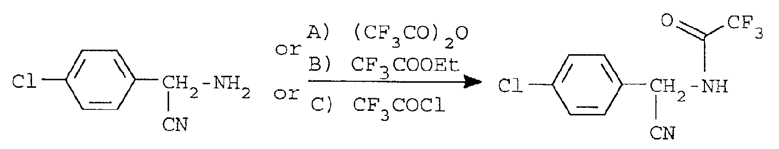 Method A: A stirred slurry of α-cyano-p-chlorobenzylamine (250g, 1.5mol) in toluene is treated with trifluoroacetic anhydride (315g, 1.5mol) at 35°C over a 90 minute period. The mixture is treated with heptane, the resultant precipitate is filtered and the filter-cake is washed with toluene/heptane to give the title product, 323.7g, 82% yield, mp 127-128°C, identified by ¹H, ¹³C and ¹⁹FNMR analyses.

Method B: A solution of α-cyano-p-chlorobenzylamine (83.3g, 0.5mol) in methanol is treated with ethyl trifluoroacetate (85.2g, 0.6mol), stirred at room temperature for about 16 hours and concentrated in vacuoto give a residue. The residue is crystallized from toluene/heptane to give the title product as a pale yellow solid, 88.3g, 67.2% yield, mp 127°- 128°C.

Method C: A mixture of a-cyano-p-chlorobenzylamine (83.3g_{;} 0.5mol) and triethylamine (50.6g, 0.5mol) in toluene is treated dropwise with trifluoroacetyl chloride (66.2g, 0.5mol), stirred at ambient temperature for about 1 hour and filtered. The filtrate is washed once with water and concentrated in vacuo to give a residue. The residue is crystallized in toluene/hexane to give the title product, 114.2g, 87% yield, mp 127°-128°C.

### EXAMPLE 2

### Preparation of N-(Arylcyanomethyl)-2,2,2-trifluoroacetamide

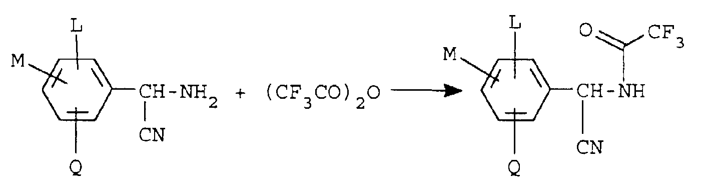

Using essentially the same procedure described as Method A in Example 1 and substituting the appropriate a-cyanobenzylamine as starting material, the following N-(arylcyanomethyl) 2,2,2-trifluoroacetamide products are obtained. The products are identified by ¹H, ¹³C and ¹⁹FNMR analyses.

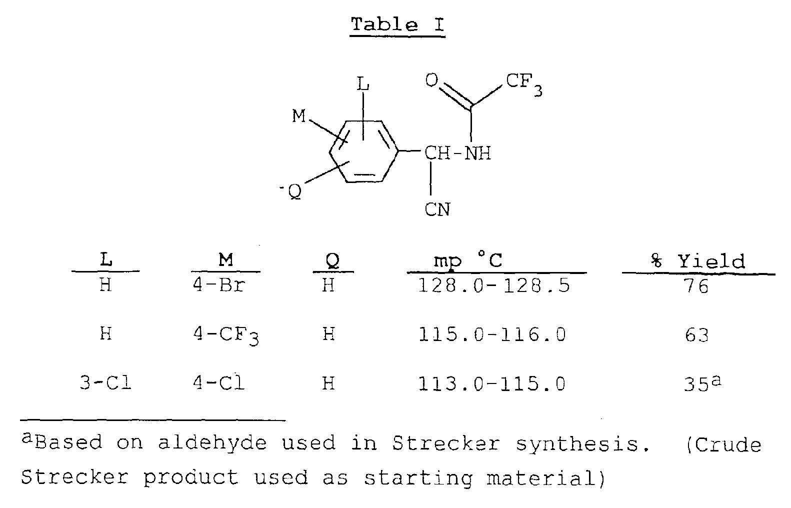

### EXAMPLE 3

### Preparation of N-(α-Cyanothienyl)-2,2,2-trifluoroacetamide

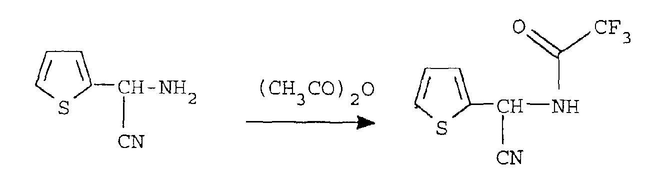

Using essentially the same procedure described as Method A in Example 1 and substituting the crude Strecker product, a-cyano-2-thiophenemethylamine, as starting material the title product is obtained in 23% yield^{a}, m.p. 73.0-74.5°C, identified by ¹H, ¹³C and ¹⁹FNMR analyses.

### EXAMPLE 4

### Preparation of N-[(p-chlorophenyl)cyanomethyl-2,2,3,3,4,4,4-heptafluorobutyramide

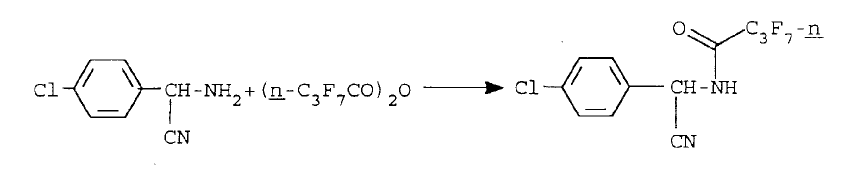

Using essentially the same procedure described as Method A in Example 1 and substituting heptafluorobutyric anhydride as the perfluoro-acylating agent, the title product is obtained as white crystals in 95% yield, mp 93.0°-95.0°C, a Based upon starting aldehyde used in Strecker synthesis.

identified by ¹H, ¹³C and ¹⁹FNMR analyses.

### EXAMPLE 5

### Preparation of N-[(p.chlorophenyl)cyanomethyll.2,2,3,3,3.pentafluoropropionamide

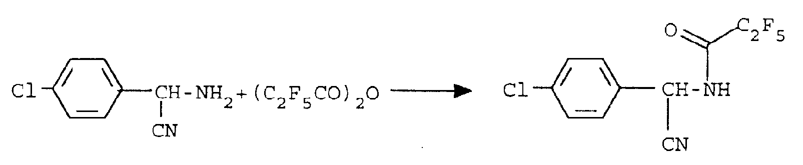

Using essentially the same procedure described as Method A in Example 1 and substituting pentafluoropropionic anhydride as the perfluoro-acylating agent, the title product is obtained as white crystals, 95% yield, mp 118.0°-118.5°C, identified by ¹H, ¹³C and ¹⁹FNMR analyses.

### EXAMPLE 6

### Preparation of 4-(p-Chlorophenyl-2-(trifluoromethyll-3-oxazolin-5-one

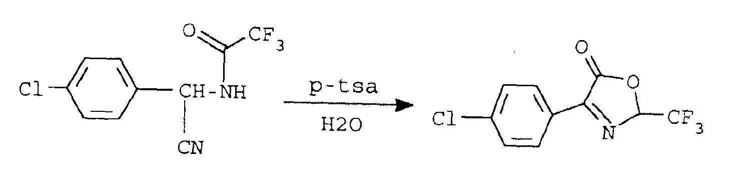

Method A: A solution of N-[(p-chlorophenyl)cyanomethyl]-2,2,2-trifluoroacetamide (0.lmol) in toluene at 80°C is treated portion-wise with p-toluene sulfonic acid monohydrate (p-tsa.H₂0)(0.11 mol) over an 0.75-1.0 hour period, stirred at 90°-95°C for 2-3 hours, cooled and filtered. The filtrate is washed twice with water and concentrated in vacuo to give an oil residue. The oil is dissolved in heptane, filtered and the filtrate is vacuum distilled to give the title product as an oil, 55.6% yield, bp 78°C/0.01 mmHg, identified by ¹H, ¹³C and ¹⁹FNMR analyses.

Method B: A solution of N-[p-(chlorophenyl)cyanomethyl]-2,2;2-trifluoroacetamide (26.3g, 0.1 mol) in toluene and methanesulfonic acid (10.7g, O.Ilmol) at 80°C is treated with water (2 mL, O.Ilmol) over a 20 minute period, stirred at 90°C for 8 hours and cooled. The reaction mixture is washed twice with water. The organic layer is concentrated in vacuo to give an oil which is vacuum distilled to give the title product as an oil 13.7g, bp 80°C/0.01 mm Hg.

### EXAMPLE 7

### Preparation of 4-(2-Thienyl-2-(trifluoromethyl)-3-oxazolin-5-one

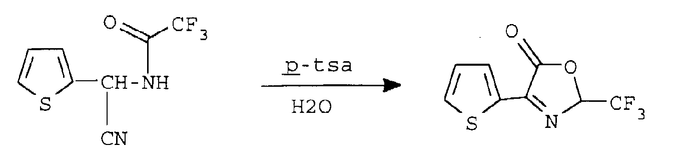

Using essentially the same procedure described as Method A in Example 6 and substituting N-(a-cyanothienyl)-2,2,2-trifluoroacetamide as starting material, the title product is obtained as a pale brown solid, 50% yield, mp 62.0°-65.0°C, identified by IR and ¹H, ¹³C and ¹⁹FNMR analyses.

### EXAMPLE 8

### Preparation of 2-perfluoroalkyl-3-oxazlin-5-one

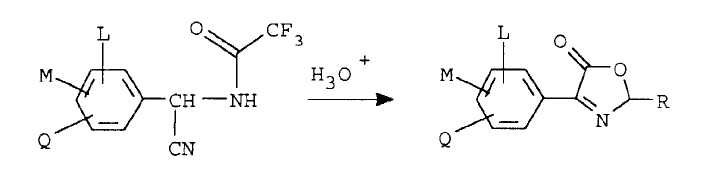

Using essentially the same procedure described as Method A in Example 6 and substituting the appropriate per- fluoroalkyanoyl aminonitrile as starting material, the compounds shown in Table ll are obtained.

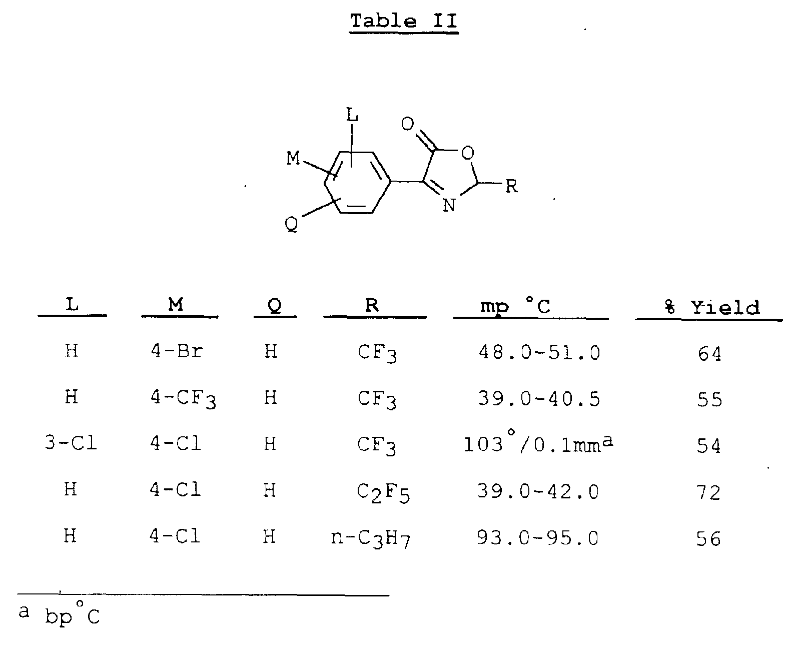

## Claims

1. A process for the preparation of a compound of formula ll 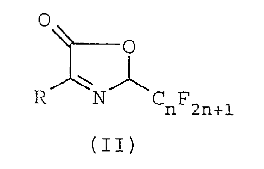 wherein
n is an integer of 1, 2, 3, 4, 5, 6, 7 or 8;
R is 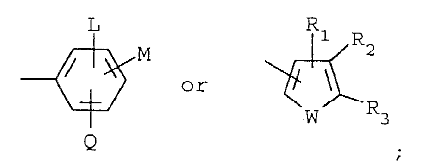 L is hydrogen or halogen;
M and Q are each independently hydrogen, halogen, CN, N0₂, C₁-C₄alkyl, C₁-C₄halpalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure 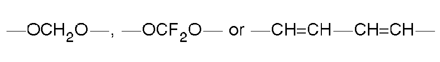 with the proviso that at least one of L, M and Q must be other than hydrogen;
R₁, R₂ and R₃ are each independently hydrogen, halogen, N0₂, CHO or R₂ and R₃ may be taken together with the atoms to which they are attached to form a ring in which R₂R₃ is represented by the structure 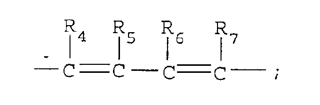 R₄, R₅, R₆ and R₇ are each independently hydrogen, halogen, CN or N0₂; and W is O or S
which comprises reacting an aminonitrile of formula lll 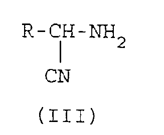 wherein R is as described above with a perfluoro-acylating agent of formula IV 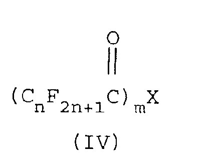 wherein m is an integer of 1 or 2, X is OR₁, Cl or O and R₁ is hydrogen or C₁-C₆alkyl with the proviso that when X is O, m must be 2 and when X is Cl or OR₁, then m must be 1 in the presence of a solvent, optionally in the presence of a base, to form a perfluoroalkanoyl aminonitrile intermediate of formula I 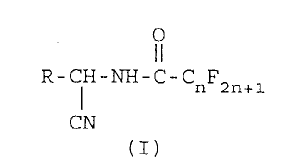 and cyclizing the formula I intermediate in the presence of an acid and at least one molar equivalent of water

2. The process according to claim 1 wherein the perfluoroacylating agent of formula IV is 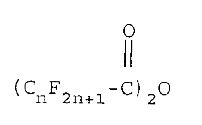 or

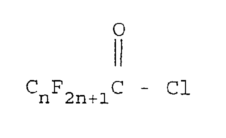

3. The process according to claim 2 wherein the perfluoroacylating agent is 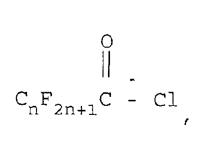 a base is present and the base is sodium or potassium carbonate or a tertiaryamine.

4. The process according to claim 1 wherein the solvent is an aromatic hydrocarbon or a halogenated aromatic hydrocarbon and the acid is sulfuric acid, methanesulfonic acid, benzenesulfonic acid, p-toluenesulfonic acid, naphthalenesulfonic acid, fluoroboric acid or a boron trifluoride complex.

5. A process for the preparation of a compound of formula ll 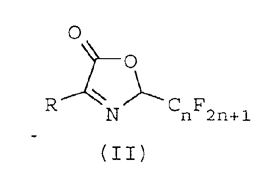 wherein n and R are as defined in claim 1 which comprises cyclizing a compound of formula I 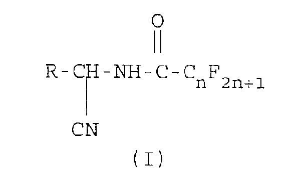 in the presence of an acid and at least one molar equivalent of water.

6. The process according to claim 5 wherein the acid is sulfuric acid methanesulfonic acid, benzenesulfonic acid,p-toluenesulfonic acid, naphthalenesulfonic acid, fluoroboric acid or a boron trifluoride complex.

7. A compound having formula I 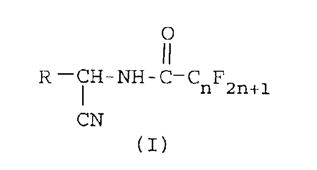 wherein n and R are as defined in claim 1.

8. The compound according to claim 7 wherein n is an integer of 1 or 2.

9. The compound according to claim 8 wherein R is 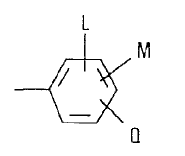 and L is hydrogen and M and Q are each independently hydrogen, halogen or C,-C₄haloalkyl.

10. The compound according to claim 8 wherein R is 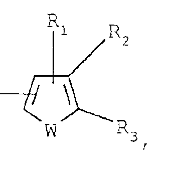 R₁ is in the 3 position and is hydrogen, R₂ and R₃ are each independently hydrogen or halogen and W is S.

## Revendications

1. Verfahren zur Herstellung einer Verbindung der Formel ll 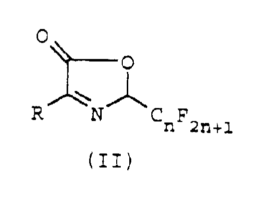 worin
n eine ganze Zahl von 1, 2, 3, 4, 5, 6, 7 oder 8 ist; R 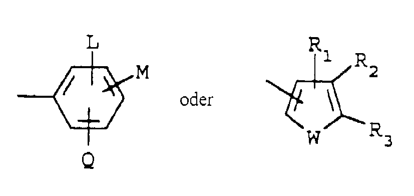 darstellt;
L Wasserstoff oder Halogen darstellt;
M und Q jeweils unabhängig Wasserstoff, Halogen, CN, NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylsulfinyl darstellen, oder, wenn M und Q sich an benach- barten Positionen befinden, sie zusammengenommen mit den Kohlenstoffatomen, an die sie gebunden sind, einen Ring bilden können, in dem MQ die Struktur -OCH₂O-, -OCF₂O- oder -CH=CH-CH=CH- wiedergibt, mit der Maßgabe, daß mindestens einer von L, M und Q keinen Wasserstoff darstellen darf;
R₁, R₂ und R₃ jeweils unabhängig Wasserstoff, Halogen; N0₂, CHO darstellen oder R₂ und R₃ zusammengenommen mit den Atomen, an die sie gebunden sind, einen Ring bilden kônnen, in dem R₂R₃ durch die Struktur 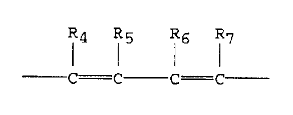 wiedergegeben werden;
R₄, R₅, R₆ und R₇ jeweils unabhängig Wasserstoff, Halogen, CN oder N0₂ darstellens und W O oder S darstellt,
umfassend Umsetzen eines Aminonitrils der Formel lll 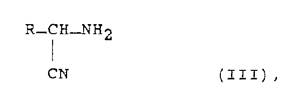 worin R wie vorstehend beschrieben ist, mit einem Perfluoracylierungsmittel der Formel IV 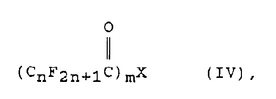 worin m eine ganze Zahl von 1 oder 2 ist, XOR₁, CI oder O darstellt und R₁ Wasserstoff oder C₁-C₆-Alkyl darstellt, mit der Maßgabe, daß, wenn X O darstellt, m 2 sein muß und wenn X CI oder OR₁ darstellt, m dann 1 sein muß, in Gegenwart eines Lösungsmittels, gegebenenfalls in Gegenwart einer Base, zur Bildung eines Perfluoralkanoyl- aminonitril-Zwischenprodukts der Formel l 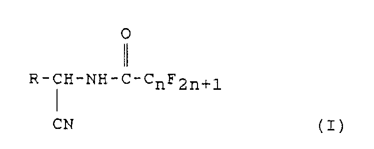 und Cyclisieren des Zwischenprodukts der Formel l in Gegenwart von einer Saure und mindestens einem Mol- äquivalent Wasser.

2. Verfahren nach Anspruch 1, wobei das Perfluoracylierungsmittel die Formel IV 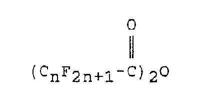 oder 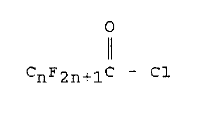 aufweist.

3. Verfahren nach Anspruch 2, wobei das Perfluoracylierungsmittel 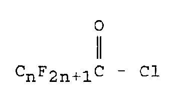 darstellt, eine Base vorliegt und die Base Natrium- oder Kaliumcarbonat oder ein tertiäres Amin darstellt.

4. Verfahren nach Anspruch 1, wobei das Lösungsmittel einen aromatischen Kohlenwasserstoff oder einen haloge- nierten aromatischen Kohlenwasserstoff darstellt und die Saure Schwefelsäure, Methansulfonsäure, Benzolsul- fonsäure, p-Toluolsulfonsâure, Naphthalinsulfonsäure, Fluorborsäure oder einen Bortrifluoridkomplex darstellt.

5. Verfahren zur Herstellung einer Verbindung der Formel ll 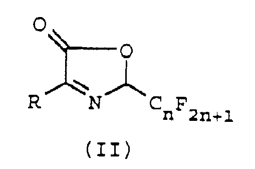 worin n und R wie in Anspruch 1 definiert sind, umfassend Cyclisieren einer Verbindung der Formel l 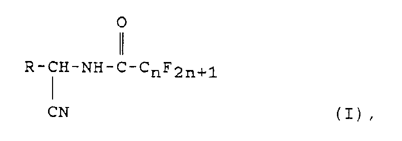 in Gegenwart einer Saure und mindestens eines Moläquivalents Wasser.

6. Verfahren nach Anspruch 5, wobei die Saure Schwefelsäure, Methansulfonsàure, Benzolsulfonsiäure, p-Toluol- sulfonsäure, Naphthalinsulfonsäure, Fluorborsäure oder ein Bortrifluoridkomplex ist.

7. Verbindung der Formel l 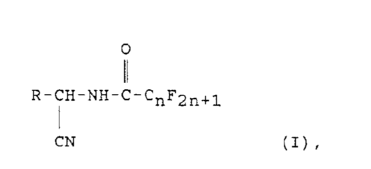 worin n und R wie in Anspruch 1 definiert sind.

8. Verbindung nach Anspruch 7, worin n eine ganze Zahl von 1 oder 2 ist.

9. Verbindung nach Anspruch 8, worin R 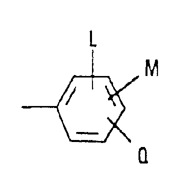 darstellt und L Wasserstoff darstellt und M und Q jeweils unabhängig Wasserstoff, Halogen oder C₁-C₄-Halogenalkyl darstellen.

10. Verbindung nach Anspruch 8, worin R 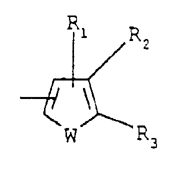 darstellt,
R₁ in der 3-Stellung vorliegt und Wasserstoff darstellt, R₂ und R₃ jeweils unabhangig Wasserstoff oder Halogen darstellen und W S darstellt.

## Patentansprüche

1. Procede de preparation d'un compose de formule ll 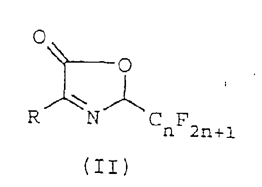 dans laquelle :
n est un entier egal ä 1, 2, 3, 4, 5, 6, 7 ou 8 ;
R represente R is 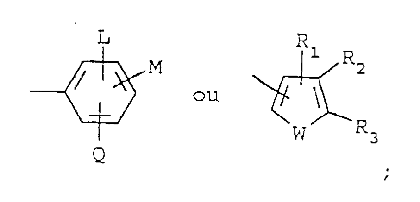 L represente un atome d'hydrogene ou un atome d'halogene ;
M et Q representent independamment chacun un atome d'hydrogene, d'halogene, un groupe CN, N0₂, alkyle en C₁-C₄, haloalkyle en C₁-C₄, alkoxy en C₁-C₄, haloalkoxy en C₁-C₄, alkylthio en C₁-C₄, alkylsulfinyle en C₁-C₄, ou lorsque M et Q sont en des positions adjacentes, ils peuvent former ensemble avec les atomes de carbone auxquels ils sont lies, un cycle dans lequel MQ represente la structure : 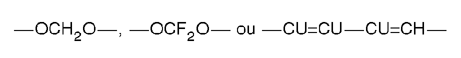 ä condition qu'au moins l'un des groupes L, M et Q soit autre qu'un atome d'hydrogene ;
R₁, R₂ et R₃ representent independamment chacun un atome d'hydrogene, d'halogene, un groupe N0₂, CHO, ou R₂ et R₃ peuvent former ensemble avec les atomes auxquels ils sont lies, un cycle dans lequel R₂R₃ est represente par la structure : 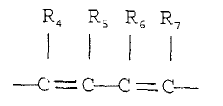 R₄, R₅, R₆ et R₇ representent independamment chacun un atome d'hydrogene, d'halogene, un groupe CN ou NO₂ ; et
W represente O ou S ; selon lequel on fait reagir un aminonitrile de formule lll : 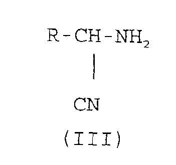 dans laquelle R est tel que decrit ci-dessus, avec un agent de perfluoroacylation de formule lV : 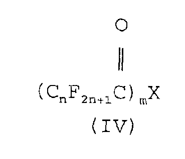 dans laquelle m est un entier egal ä 1 ou 2, X represente OR₁, Cl ou O, et R₁ represente un atome d'hydrogene ou un groupe alkyle en C₁-C₆, ä condition que lorsque X represente O, m soit egal ä 2, et que lorsque X represente Cl ou OR₁, m soit egal ä 1, en presence d'un solvant, eventuellement en presence d'une base, afin de former un intermediaire de type perfluoroalcanoyl aminonitrile de formule l : 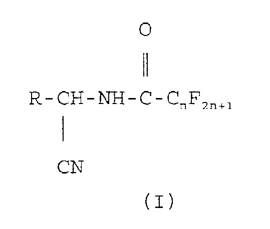 et on cyclise l'intermédiaire de formule l en presence d'un acide et d'au moins un equivalent molaire d'eau.

2. Procede selon la revendication 1, dans lequel l'agent de perfluoroacylation de formule lV est : 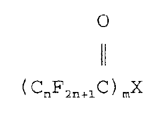 ou

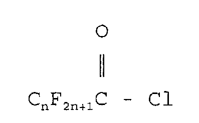

3. Procede selon la revendication 2, dans lequel l'agent de perfluoroacylation, est: 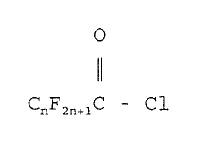 une base etant presente, et la base etant le carbonate de sodium ou de potassium, ou une amine tertiaire.

4. Procede selon la revendication 1, dans lequel le solvant est un hydrocarbure aromatique ou un hydrocarbure aromatique halogene, et l'acide est l'acide sulfurique, l'acide methanesulfonique, l'acide benzenesulfonique, l'acide p-toluenesulfonique, l'acide naphtalenesulfonique, l'acide fluoroborique ou un complexe de trifluorure de bore.

5. Procede de preparation d'un composé de formule ll 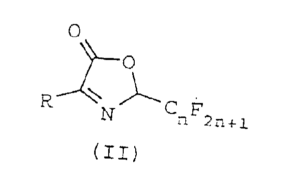 dans laquelle :
n et R sont tels que definis dans la revendication 1, selon lequel on cyclise un compose de formule l : 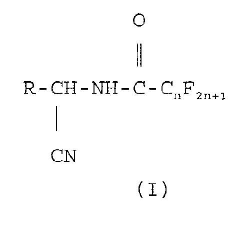 en presence d'un acide et d'au moins un equivalent molaire d'eau.

6. Procede selon la revendication 5, dans lequel l'acide est l'acide sulfurique, l'acide methanesulfonique, l'acide benzenesulfonique, l'acide p-toluènesulfonique, l'acide naphtalenesulfonique, l'acide fluoroborique ou un complexe de trifluorure de bore.

7. Compose de formule l : 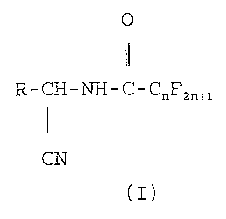 dans laquelle n et R sont tels que definis dans la revendication 1.

8. Compose selon la revendication 7, dans lequel n est un entier egal ä 1 ou 2.

9. Compose selon la revendication 8, dans lequel R represente : R is 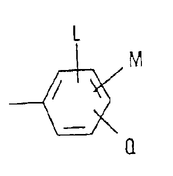 et L represente un atome d'hydrogene, et M ainsi que Q representent independamment chacun un atome d'hy- drogene, d'halogene ou un groupe haloalkyle en C₁-C₄-

10. Compose selon la revendication 8, dans lequel R represente : R is 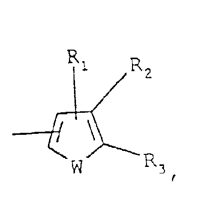 R₁ est en position 3 et represente un atome d'hydrogene, R₂ et R₃ representent independamment chacun un atome d'hydrogene ou d'halogene, et W represente S.
